(19) Europäisches Patentamt
European Patent Office
Office européen des brevets

(11) Veröffentlichungsnummer: **0 300 312**
**A2**

(12) # EUROPÄISCHE PATENTANMELDUNG

(21) Anmeldenummer: **88111035.7**

(22) Anmeldetag: **11.07.88**

(51) Int. Cl.4: **C07D 207/337 , C07D 405/04 , C07D 409/04 , A01N 43/36**

(30) Priorität: **24.07.87 DE 3724554**

(43) Veröffentlichungstag der Anmeldung: **25.01.89 Patentblatt 89/04**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Wollweber, Detlef, Dr.**
**Paul-Ehrlich-Strasse 17**
**D-5600 Wuppertal 1(DE)**
Erfinder: **Brandes, Wilhelm, Dr.**
**Eichendorffstrasse 3**
**D-5653 Leichlingen(DE)**

(54) **Substituierte 3-Arylpyrrole.**

(57) Substituierte 3-Arylpyrrole der allgemeinen Formel (I),

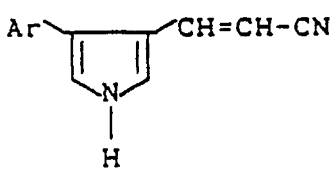

(I)

in welcher
Ar für gegebenenfalls substituiertes Aryl oder für gegebenenfalls substituiertes Heteroaryl steht,
deren Isomere und Isomerengemische sowie ihre Verwendung zur Bekämpfung von Schädlingen.

Die erfindungsgemäßen Verbindungen der Formel (I) können nach bekannten Verfahren hergestellt werden, indem man geeignete 3-Aryl-pyrrol-4-aldehyde entweder mit geeigneten Triphenylphosphoniumsalzen oder mit geeigneten Phosphonsäureestern umsetzt.

EP 0 300 312 A2

## Substituierte 3-Arylpyrrole

Die Erfindung betrifft neue substituierte 3-Arylpyrrole, ein Verfahren zu ihrer Herstellung sowie ihre Verwendung als Schädlingsbekämpfungsmittel.

Es ist bereits bekannt, daß bestimmte substituierte 3-Arylpyrrole, wie beispielsweise das 3-(2,3-Dichlorphenyl)-4-cyano-pyrrol fungizide Eigenschaften besitzen (vgl. EP 174 910 und EP 182 738).

Die Wirksamkeit dieser vorbekannten Verbindungen ist jedoch, insbesondere bei niedrigen Aufwandmengen und Konzentrationen nicht in allen Anwendungsgebieten völlig zufriedenstellend. Problematisch ist bei Vertretern dieser Stoffklasse mitunter auch die Pflanzenverträglichkeit.

Es wurden neue substituierte 3-Arylpyrrole der allgemeinen Formel (I),

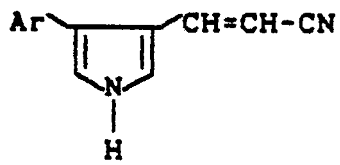

(I)

in welcher
Ar für gegebenenfalls substituiertes Aryl oder für gegebenenfalls substituiertes Heteroaryl steht,
gefunden.

Die Verbindungen der Formel (I) können als geometrische Isomere oder Isomerengemische unterschiedlicher Zusammensetzung vorliegen. Sowohl die reinen Isomeren als auch die Isomerengemische werden Erfindungsgemäß beansprucht.

Weiterhin wurde gefunden, daß man die neuen substituierten 3-Arylpyrrole der allgemeinen Formel (I),

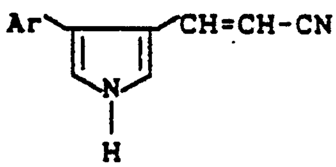

(I)

in welcher
Ar für gegebenenfalls substituiertes Aryl oder für gegebenenfalls substituiertes Heteroaryl steht,
erhält, wenn man 3-Aryl-pyrrol-4-aldehyde der Formel (II),

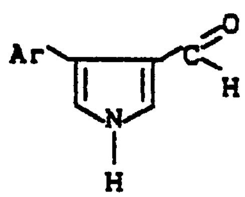

(II)

in welcher
Ar die oben angegebene Bedeutung hat,
entweder

(a) mit Triphenylphosphoniumsalzen der Formel (III),

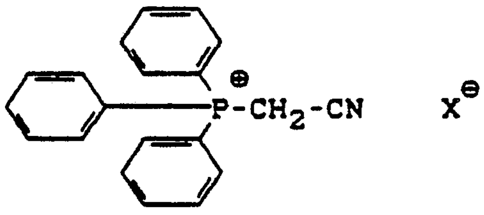

(III)

in welcher
$X^{\ominus}$ für ein geeignetes Gegenion steht,
oder

(b) mit Phosphonsäureestern der Formel (IV),

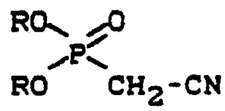

(IV)

in welcher
R für Alkyl steht, jeweils in Gegenwart einer Base und in Gegenwart eines Verdünnungsmittels umsetzt.

Schließlich wurde gefunden, daß die neuen substituierten 3-Arylpyrrole der allgemeinen Formel (I) eine gute Wirkung gegen Schädlinge besitzen.

Überraschenderweise zeigen die erfindungsgemäßen substituierten 3-Aryl-pyrrole der allgemeinen Formel (I) eine bessere Wirksamkeit gegenüber pilzlichen Pflanzenschädlingen als die aus dem Stand der Technik bekannten substituierten 3-Arylpyrrole, wie beispielsweise das 3-(2,3-Dichlorphenyl)-4-cyano-pyrrol, welche chemisch und wirkungsmäßig naheliegende Verbindungen sind und darüberhinaus weisen sie eine erheblich bessere Pflanzenverträglichkeit auf.

Die erfindungsgemäßen substituierten 3-Arylpyrrole sind durch die Formel (I) allgemein definiert. Bevorzugt sind Verbindungen der Formel (I), bei welchen
Ar für jeweils gegebenenfalls einfach bis mehrfach, gleich oder verschieden durch Halogen, Nitro oder geradkettiges oder verzweigtes Alkyl mit 1 bis 4 Kohlenstoffatomen substituiertes Pyridyl, Furyl oder Thienyl steht oder für gegebenenfalls einfach bis mehrfach, gleich oder verschieden substituiertes Phenyl steht, wobei als Substituenten infrage kommen: Halogen, Cyano, Nitro, jeweils geradkettiges oder verzweigtes Alkyl, Alkoxy, Alkylthio oder Alkoxycarbonyl mit jeweils 1 bis 6 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, Phenyl sowie zweifach verknüpftes, gegebenenfalls durch Fluor substituiertes Dioxyalkylen mit 1 oder 2 Kohlenstoffatomen.

Besonders bevorzugt sind Verbindungen der Formel (I), bei welchen
Ar für jeweils gegebenenfalls einfach, zweifach oder dreifach, gleich oder verschieden durch Fluor, Chlor, Brom, Methyl und Ethyl substituiertes 2-Pyridyl, 3-Pyridyl, 4-Pyridyl, 3-Furyl, 2-Furyl, 3-Thienyl oder 2-Thienyl steht oder für gegebenenfalls ein- bis dreifach, gleich oder verschieden substituiertes Phenyl steht, wobei als Substituenten infrage kommen: Fluor, Chlor, Brom, Methyl, Ethyl, n- oder i-Propyl, Methoxy, Ethoxy, Methylthio, Trifluormethyl, Trifluormethoxy, Trifluormethylthio, Cyano, Nitro, Phenyl, Dioxymethylen und Dioxydifluormethylen.

Ganz besonders bevorzugt sind Verbindungen der Formel (I), bei welchen
Ar für gegebenenfalls einfach oder zweifach, gleich oder verschieden substituiertes Phenyl steht, wobei als Substituenten infrage kommen: Fluor, Chlor, Brom, Methyl, Ethyl, Methoxy, Ethoxy, Methylthio, Trifluormethyl, Trifluormethoxy, Trifluormethylthio, Nitro und Dioxydifluormethylen; oder für jeweils gegebenenfalls ein- oder zweifach durch Chlor und/oder Methyl substituiertes 2-Pyridyl, 4-Pyridyl, 2-Furyl oder 2-Thienyl steht.

Im einzelnen seien außer den bei den Herstellungsbeispielen genannten Verbindungen die folgenden substituierten 3-Arylpyrrole der allgemeinen Formel (I) genannt:

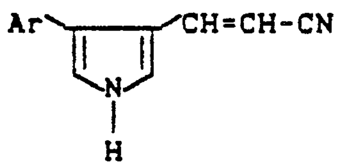

(I)

| Ar | Ar |
|---|---|
| 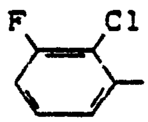 | 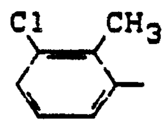 |
| 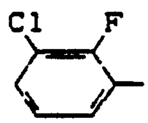 | 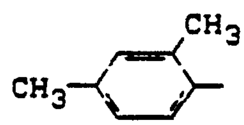 |

| Ar | Ar |
|---|---|
| 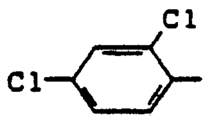 | 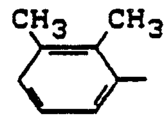 |
| 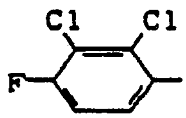 | 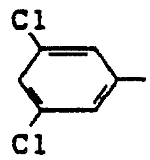 |
| 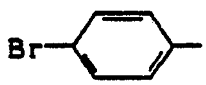 | 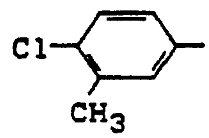 |
| 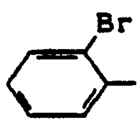 | 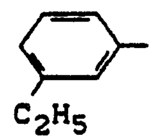 |
| 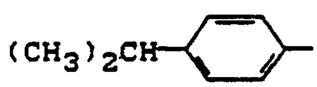 | 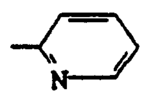 |
| 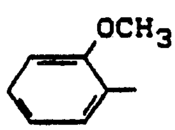 | 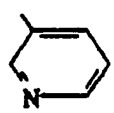 |
| 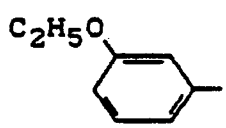 | 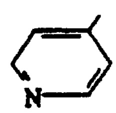 |

| Ar | Ar |
|---|---|
| 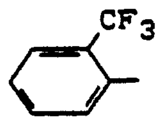 | 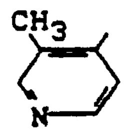 |
| 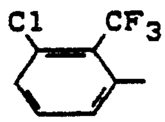 | 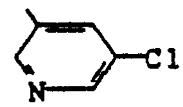 |
| 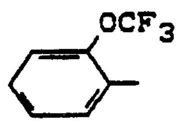 | 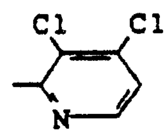 |
| 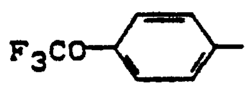 | 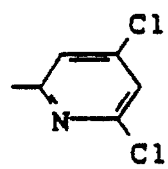 |
| 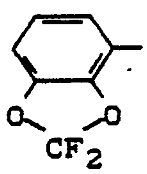 | 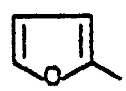 |
| 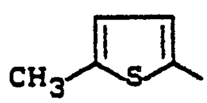 | 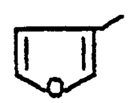 |
| 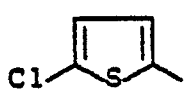 | 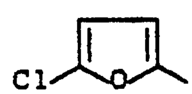 |

| Ar | Ar |
|---|---|
| 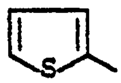 | 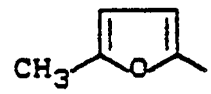 |
| 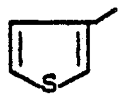 | |

Verwendet man beispielsweise 3-(2-Chlorphenyl)-pyrrol-4-aldehyd und Triphenylcyanmethylphosphoniumchlorid als Ausgangsstoffe, so läßt sich der Reaktionsablauf des erfindungsgemäßen Verfahrens (a) durch das folgende Formelschema darstellen:

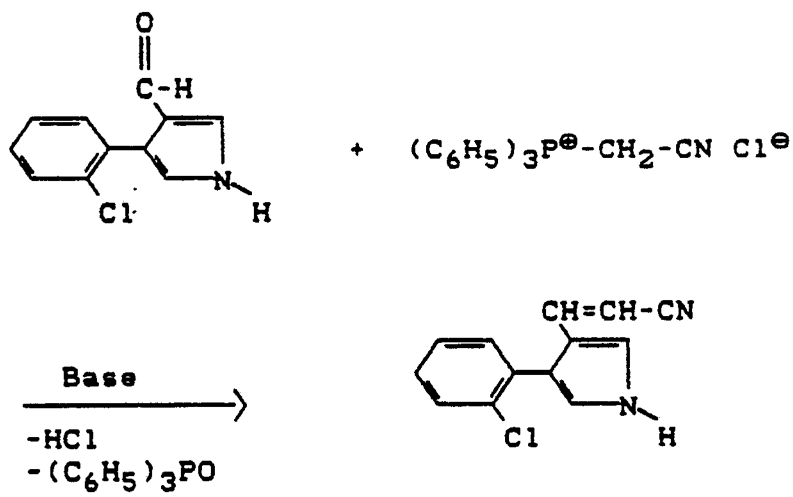


Verwendet man beispielsweise 3-(2-Methylphenyl)-pyrrol-4-aldehyd und Cyanmethylphosphonsäurediethylester als Ausgangsstoffe, so läßt sich der Reaktionsablauf des erfindungsgemäßen Verfahrens (b) durch das folgende Formelschema darstellen:

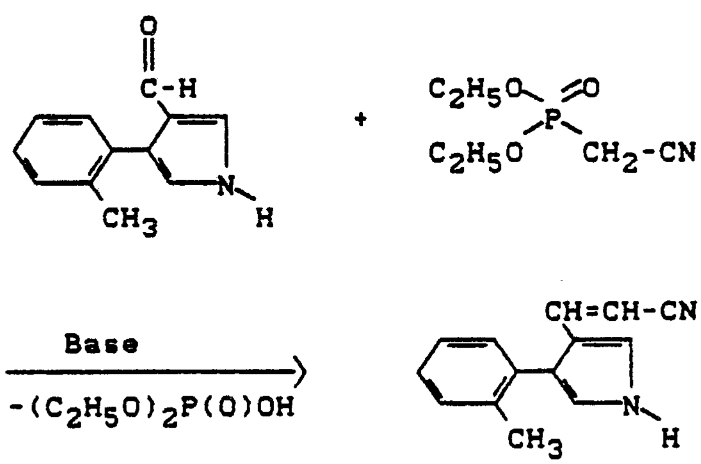


Die zur Durchführung der erfindungsgemäßen Verfahren (a) und (b) als Ausgangsstoffe benötigten 3-Arylpyrrol-4-aldehyde sind durch die Formel (II) allgemein definiert. In dieser Formel (II) steht Ar vorzugsweise für diejenigen Reste, die bereits im Zusammenhang mit der Beschreibung der erfindungsgemäßen Stoffe der Formel (I) als bevorzugt für diesen Substituenten genannt wurden.

Die 3-Aryl-pyrrol-4-aldehyde der Formel (II) sind bekannt (vgl. z.B. EP 174 910) oder erhältlich in Analogie zu bekannten Verfahren, beispielsweise wenn man 4-Cyano-3-aryl-pyrrole der Formel (V),

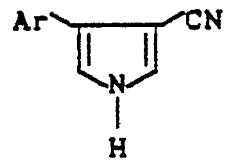

(V)

in welcher

Ar die oben angegebene Bedeutung hat,

mit Reduktionsmitteln wie beispielsweise Diisobutylaluminiumhydrid, gegebenenfalls in Gegenwart eines Verdünnungsmittels wie beispielsweise Toluol bei Temperaturen zwischen - 20 °C und + 80 °C umsetzt.

Die 4-Cyano-3-aryl-pyrrole der Formel (V) sind bekannt (vgl. z.B. EP 174 910 oder EP 182 738).

Die zur Durchführung des erfindungsgemäßen Verfahrens (a) als Ausgangsstoffe benötigten Triphenylphosphoniumsalze sind durch die Formel (III) allgemein definiert. In dieser Formel (III) steht X vorzugsweise für Halogen, insbesondere für Chlor oder Brom.

Die Triphenylphosphoniumsalze der Formel (III) sind allgemein bekannte Verbindungen der organischen Chemie.

Die zur Durchführung des erfindungsgemäßen Verfahrens (b) als Ausgangsstoffe benötigten Phosphonsäureester sind durch die Formel (IV) allgemein definiert. In dieser Formel (IV) steht R vorzugsweise für geradkettiges oder verzweigtes Alkyl mit 1 bis 4 Kohlenstoffatomen, insbesondere für Methyl oder Ethyl.

Die Phosphonsäureester der Formel (IV) sind ebenfalls allgemein bekannte Verbindungen der organischen Chemie.

Als Verdünnungsmittel zur Durchführung der erfindungsgemäßen Verfahren (a) und (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Die erfindungsgemäßen Verfahren (a) und (b) werden vorzugsweise in Gegenwart einer geeigneten Base durchgeführt. Als solche kommen alle üblicherweise verwendbaren aprotischen, anorganischen und organischen Basen in Frage. Vorzugsweise verwendet man Alkalimetallhydride, -amide oder -alkoholate, wie beispielsweise Natriumhydrid, Natriumamid, Natriummethylat, Natriumethylat, Kalium-t-butylat oder auch metallorganische Verbindungen, wie beispielsweise n-Butyllithium.

Die Reaktionstemperaturen können bei der Durchführung der erfindungsgemäßen Verfahren (a) und (b) in einem größeren Bereich variiert werden. Im allgemeinen arbeitet man bei Temperaturen zwischen - 20 °C und + 120 °C, vorzugsweise bei Temperaturen zwischen 0 °C und 80 °C.

Zur Durchführung des erfindungsgemäßen Verfahrens (a) setzt man pro Mol an 3-Aryl-pyrrol-4-aldehyd der Formel (II) im allgemeinen 1.0 bis 3.0 Mol, vorzugsweise 1.0 bis 2.0 Mol an Triphenylphosponiumsalz der Formel (III) und 1.0 bis 3.0 Mol, vorzugsweise 1.0 bis 2.0 Mol an Base ein.

Die Reaktionsdurchführung, Aufarbeitung und Isolierung der Reaktionsprodukte erfolgt nach allgemein üblichen Methoden (vgl. z.B. "Organikum"; 15. Auflage, S. 494: VEB Deutscher Verlag der Wissenschaften, Berlin 1981 sowie die Herstellungsbeispiele).

Zur Durchführung des erfindungsgemäßen Verfahrens (b) setzt man pro Mol an 3-Aryl-pyrrol-4-aldehyd der Formel (II) im allgemeinen 1.0 bis 3.0 Mol, vorzugsweise 1.0 bis 2.0 Mol an Phosphonsäureester der Formel (IV) und gegebenenfalls 1.0 bis 3.5 Mol, vorzugsweise 1.0 bis 2.5 Mol an Base ein.

In einer bevorzugten Ausführungsform ist es möglich, den als Ausgangsverbindung eingesetzten Phosphonsäureester der Formel (IV) in einer vorgelagerten Reaktion aus Trialkylphosphit und Halogenacetonitril herzustellen und direkt aus der Reaktionsmischung heraus weiter umzusetzen gemäß dem erfindungsgemäßen Verfahrens (b).

Die Reaktionsdurchführung, Aufarbeitung und Isolierung der Reaktionsprodukte erfolgt nach allgemein üblichen Methoden (vgl. z.B. Synthesis 1969, 170 oder Synthesis 1977, 126 sowie die Herstellungsbeispiele).

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide eingesetzt.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Dabei lassen sich die erfindungsgemäßen Wirkstoffe mit besonders gutem Erfolg zur Bekämpfung von Krankheiten im Obst- und Gemüsebau, wie beispielsweise gegen den Erreger des Bohnengrauschimmels (Botrytis cinerea) oder zur Bekämpfung von Reiskrankheiten, wie beispielsweise gegen den Erreger der Reisfleckenkrankheit (Pyricularia oryzae) einsetzen.

Daneben zeigen die erfindungsgemäßen Wirkstoffe eine gute in vitro-Wirkung im Mycelwachstumstest.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoff, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage; z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B.

gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Moybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsgformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-% vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

## Herstellungsbeispiele

### Beispiel 1

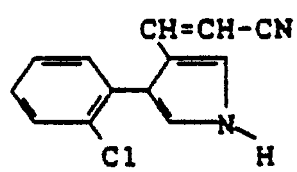

(Verfahen a)

Zu 16,8 g (0,0498 Mol) Cyanmethyltriphenylphosphoniumchlorid in 30 ml Toluol gibt man bei Raumtemperatur unter einer Argonschutzgasatmosphäre unter Rühren tropfenweise 31,1 ml (0,0498 Mol) n-Butyllithium in Hexan (Konzentration 1,6 Mol/1), rührt nach beendeter Zugabe 1 Stunde nach und gibt dann tropfenweise unter Rühren 5,1 g (0,0249 Mol) 3-(2-Chlorphenyl)-pyrrol-4-aldehyd in 120 ml Toluol/Tetrahydrofuran-Mischung (1 : 3) zu, rührt nach beendeter Zugabe 12 Stunden bei 50 °C, kühlt dann ab auf Raumtemperatur, filtriert, verdünnt das Filtrat mit Essigester, wäscht mit Wasser, trocknet über Natriumsulfat und entfernt das Lösungsmittel im Vakuum. Das so erhältliche E/Z-Isomerengemisch läßt sich

durch Chromatographie an Kieselgel (Laufmittel Essigester / Cyclohexan 1 : 5 ) auftrennen.

Man erhält 2,3 g (41 % der Theorie) an (Z)-β-[3-(2-Chlorphenyl)-pyrrol-4-yl]-acrylnitril vom Schmelzpunkt 145 °C - 146 °C und 1,9 g (34 % der Theorie ) an (E)-β-[3-(2-Chlorphenyl)-pyrrol-4-yl]-acrylnitril vom Schmelzpunkt 142 °C - 143 °C.

Herstellung der Ausgangsverbindung

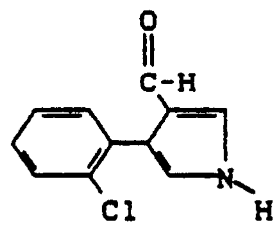

Zu 15 g (0,0743 Mol) 3-(2-Chlorphenyl)-pyrrol-4-carbonitril in 300 ml absolutem Toluol gibt man bei - 20 °C unter einer Argonschutzgasatmosphäre tropfenweise unter Rühren 99 ml (0,1485 Mol) einer 1.5-molaren Diisobutylaluminiumhydridlösung in Toluol, rührt nach beendeter Zugabe 4 Stunden bei - 20 °C nach, läßt die Mischung auf Raumtemperatur kömmen, hydrolysiert mit 400 ml 10prozentiger wässriger Zitronensäurelösung, trennt die organische Phase ab, wäscht die wässrige Phase mit Ether, trocknet die vereinigkten organischen Phasen über Natriumsulfat, engt im Vakuum ein und chromatographiert den Rückstand an Kieselgel (Laufmittel Essigester / Cyclohexen 2 : 1).

Man erhält 11,8 g (78 % der Theorie) an 3-(2-Chlorphenyl)-pyrrol-4-aldehyd vom Schmelzpunkt 147 °C -148 °C.

Beispiel 2

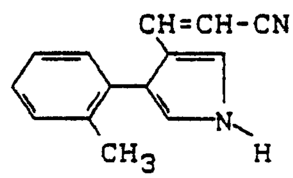

(Verfahren b)

2,5 g (0,0326 Mol) Chloracetonitril und 5,4 g (0,0326 Mol) Triethylphosphit werden so lange auf 200 °C erhitzt, bis die Gasentwicklung beendet ist. Zu der erkalteten Mischung gibt man 30 ml Dimethylformamid und 1,8 g (0,034 Mol) Natriummethylat und anschließend bei 0 °C tropfenweise unter Rühren 3,0 g (0,0162 Mol) 3-(2-Methylphenyl)-pyrrol-4-aldehyd in 5 ml Dimethylformamid. Nach beendeter Zugabe rührt man 16 Stunden bei Raumtemperatur, gibt dann die Reaktionsmischung in Wasser, extrahiert mit Essigester, wäscht mit Wasser, trocknet über Natriumsulfat, engt im Vakuum ein und reinigt den Rückstand durch Chromatographie an Kieselgel (Laufmittel Essigester / Cyclohexan 5 : 1).

Man erhält 0,5 g (15 % der Theorie) an (Z)-β-[3-(2-Methylphenyl)-pyrrol-4-yl]-acrylnitril vom Schmelzpunkt 74 °C - 75 °C und 1,2 g (36 % der Theorie) an (E)-β-[3-(2-Methylphenyl)-pyrrol-4-yl]-acrylnitril vom Schmelzpunkt 152 °C - 154 °C.

In entsprechender Weise und gemäß den allgemeinen Angaben zur Herstellung erhält man die folgenden substituierten 3-Aryl-pyrrole der allgemeinen Formel (I):

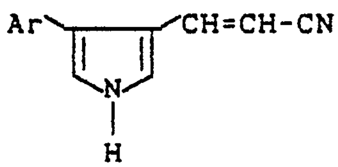

(I)

| Bsp. Nr. | Ar | Schmelzpunkt/° C |
|---|---|---|
| 3 | 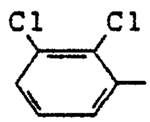 | 153-154 (Z-Form) |
| 4 | 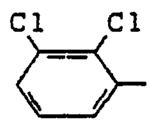 | 187-188 (E-Form) |
| 5 | 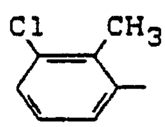 | 140-141 (Z-Form) |
| 6 | 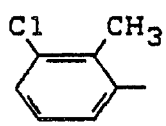 | 152-154 (E-Form) |
| 7 | 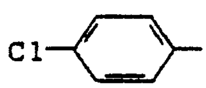 | 154 |

Anwendungsbeispiele

In dem folgenden Anwendungsbeispiel wurde die nachstehend aufgeführte Verbindung als Vergleichsubstanz eingesetzt:

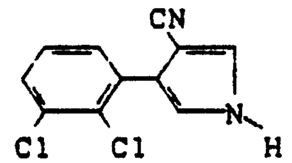

(A)

3-(2,3-Dichlorphenyl)-pyrrol-4-carbonitril

(bekannt aus EP 174 910)

Beispiel A

Botrytis-Test (Bohne)/protektiv
Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

Eine deutliche Überlegenheit in Wirksamkeit gegenüber dem Stand der Technik zeigen in diesem Test z.B. die Verbindungen gemäß den Herstellungsbeispielen: 1, 3 und 4.

## Ansprüche

1. Substituierte 3-Arylpyrrole der allgemeinen Formel (I),

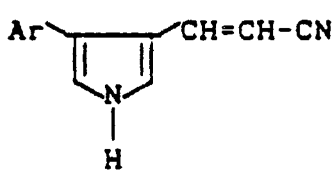

(I)

in welcher
Ar für gegebenenfalls substituiertes Aryl oder für gegebenenfalls substituiertes Heteroaryl steht,
deren Isomere und Isomerengemische.

2. Substituierte 3-Arylpyrrole gemäß Anspruch 1, worin in der Formel (I)
Ar für jeweils gegebenenfalls einfach bis mehrfach, gleich oder verschieden durch Halogen, Nitro oder geradkettiges oder verzweigtes Alkyl mit 1 bis 4 Kohlenstoffatomen substituiertes Pyridyl, Furyl oder Thienyl steht oder für gegebenenfalls einfach bis mehrfach, gleich oder verschieden substituiertes Phenyl

steht, wobei als Substituenten infrage kommen: Halogen, Cyano, Nitro, jeweils geradkettiges oder verzweigtes Alkyl, Alkoxy, Alkylthio oder Alkoxycarbonyl mit jeweils 1 bis 6 Kohlenstoffatomen, jeweils geradkettiges oder ver zweigtes Halogenalkyl, Halogenalkoxy oder Halogenalkylthio mit jeweils 1 bis 4 Kohlenstoffatomen und 1 bis 9 gleichen oder verschiedenen Halogenatomen, Phenyl sowie zweifach verknüpftes gegebenenfalls durch Fluor substituiertes Dioxyalkylen mit 1 oder 2 Kohlenstoffatomen, deren Isomere und Isomerengemische.

3. Substituierte 3-Arylpyrrole gemäß Anspruch 1, worin in der Formel (I)
Ar für jeweils gegebenenfalls einfach, zweifach oder dreifach, gleich oder verschieden durch Fluor, Chlor, Brom, Methyl und Ethyl substituiertes 2-Pyridyl, 3-Pyridyl, 4-Pyridyl, 3-Furyl, 2-Furyl, 3-Thienyl oder 2-Thienyl steht oder für gegebenenfalls ein- bis dreifach, gleich oder verschieden substituiertes Phenyl steht, wobei als Substituenten infrage kommen: Fluor, Chlor, Brom, Methyl, Ethyl, n- oder i-Propyl, Methoxy, Ethoxy, Methylthio, Trifluormethyl, Trifluormethoxy, Trifluormethylthio, Cyano, Nitro, Phenyl, Dioxymethylen und Dioxydifluormethylen und
deren Isomere und Isomerengemische.

4. Substituierte 3-Arylpyrrole gemäß Anspruch 1, worin in der Formel (I)
Ar für gegebenenfalls einfach oder zweifach, gleich oder verschieden substituiertes Phenyl steht, wobei als Substituenten infrage kommen: Fluor, Chlor, Brom, Methyl, Ethyl, Methoxy, Ethoxy, Methylthio, Trifluormethyl, Trifluormethoxy, Trifluormethylthio, Nitro und Dioxydifluormethylen; oder für jeweils gegebenenfalls ein- oder zweifach durch Chlor und/oder Methyl substituiertes 2-Pyridyl, 4-Pyridyl, 2-Furyl oder 2-Thienyl steht und deren Isomere und Isomerengemische.

5. Verfahren zur Herstellung von substituierten 3-Arylpyrrolen der allgemeinen Formel (I),

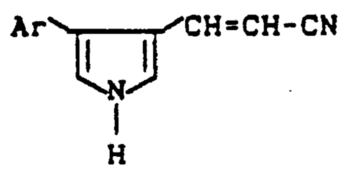

(I)

in welcher
Ar für gegebenenfalls substituiertes Aryl oder für gegebenenfalls substituiertes Heteroaryl steht,
deren Isomere und Isomerengemische, dadurch gekennzeichnet, daß man 3-Aryl-pyrrol-4-aldehyde der Formel (II),

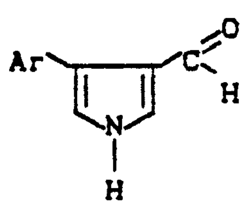

(II)

in welcher
Ar die oben angegebene Bedeutung hat,
entweder (a) mit Triphenylphosphoniumsalzen der Formel (III),

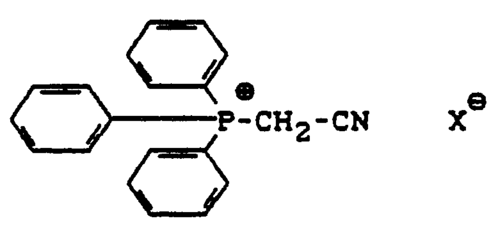

(III)

in welcher
$X^{\ominus}$ für ein geeignetes Gegenion steht,
oder
(b) mit Phosphonsäureestern der Formel (IV),

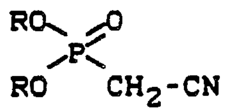

(IV)

in welcher
R für Alkyl steht,
jeweils in Gegenwart einer Base und in Gegenwart eines Verdünnungsmittels umsetzt.

6. Schädlingsbekämpfungsmittel, gekennzeichnet durch einen Gehalt an mindestens einem substituierten 3-Arylpyrrol der Formel (I) nach den Ansprüchen 1 und 5.

7. Verwendung von substituierten 3-Arylpyrrolen der Formel (I) nach den Ansprüchen 1 und 5 zur Bekämpfung von Schädlingen.

8. Verfahren zur Bekämpfung von Schädlingen, dadurch gekennzeichnet, daß man ein substituiertes 3-Arylpyrrol der Formel (I) nach den Ansprüchen 1 und 5 auf Schädlinge und/oder ihren Lebensraum einwirken läßt.

9. Verfahren zur Herstellung von Schädlingsbekämpfungsmitteln, dadurch gekennzeichnet, daß man substituierte 3-Arylpyrrole der Formel (I) nach den Ansprüchen 1 und 5 mit Streckmitteln und/oder oberflächenaktiven Mitteln vermischt.